# EUROPEAN PATENT APPLICATION

(11) **EP 2 305 305 A1**
(43) Date of publication of application: **06.04.2011**
(21) Application number: 09750557.2
(22) Date of filing: 19.05.2009
(51) Int. Cl.: A61K 45/00, A61K 31/137, A61K 31/42, A61K 31/519, A61K 31/7056, A61K 38/00, A61P 1/04, A61P 11/06, A61P 13/12, A61P 17/06, A61P 19/02, A61P 21/04, A61P 25/00, A61P 29/00, A61P 37/02, A61P 37/08, A61P 43/00

(54) **AGENT FOR INDUCTION/MAINTENANCE OF REMISSION**

(30) Priority: 20.05.2008 JP 2008131634
(71) Applicant: Kyorin Pharmaceutical Co., Ltd., Tokyo 101-8311 (JP)
(72) Inventor: KURIYAMA, Kazuhiko, Shimotsuga-gun Tochigi 329-0114 (JP); KAGO, Tomoyuki, Shimotsuga-gun Tochigi 3290114 (JP); YASUE, Tokutaro, Shimotsuga-gun Tochigi 329-0114 (JP)
(74) Representative: Vossius & Partner
(86) International application number: PCT/JP2009/059176
(87) International publication number: WO 2009/142195

(57) **Abstract**

Disclosed is a method for maintaining induced remission of an immune disease, which can alleviate or reduce any serious side effect and the burdens exerted on a patient suffering from the same. The method comprises carrying out the remission induction for an immune disease by the use of a biological agent or a nucleic acid synthesis-inhibitory agent and then using an agonist for sphingosine-1-phosphate receptor to the patient.

## Description

### Technical Field

The present invention relates to a remission-maintaining therapeutic agent for immune diseases and a therapeutic method for maintaining the remission thereof.

### Background Art

The treatment of immune diseases such as rheumatoid arthritis has recently undergone a considerable transfiguration and the strategy of treatment, in itself, has been reconstructed. Such transfiguration has really been caused by the repletion of antirheumatic agents, showing excellent clinical effects, such as methotrexate and the introduction of biological preparations showing the epoch-making clinical effects and the effects of inhibiting any joint-breakage. However, it would almost be impossible to discontinue the treatment after the remission of such a disease and accordingly, the administration of these agents should be continued even after the remission induction therapy under the present conditions. Moreover, the antirheumatic agents such as methotrexate may often produce serious side effects and therefore the patients suffering from such a disease should regularly undergo any medical examination. On the other hand, the biological agents are in general administered intravenously or subcutaneously and therefore, the use thereof is not preferred in view of the quality of life (QOL) of patients.
Incidentally, Patent Document 1 discloses the use of a diaryl sulfide or diaryl ether compound having a 2-amino-1,3-propanediol structure for the treatment of the adjuvant-induced arthritis.
In Patent Document 1, however, the diaryl sulfide or diaryl ether compound having a 2-amino-1,3-propanediol structure is used in combination with an anti-inflammatory agent such as methotrexate from the beginning and Patent Document 1 does not disclose, at all, such an idea that the anti-inflammatory agent is switched over to the diaryl sulfide compound or the diaryl ether compound in order to maintain the remitted conditions even after the achievement of the remission of an intended immune disease.

### Prior Art Literature

### Patent Document:

Patent Document 1: WO 2006/009092

### Summary of the Invention

### Problems That the Invention is to Solve

It is accordingly an object of the present invention to provide a method for the maintenance of the induced remission of an immune disease, which can significantly reduce any serious side effect and likewise reduce the burdens exerted on the patients.

### Means for the Solution of the Problems

The inventors of this invention have conducted intensive studies to achieve the foregoing object of the present invention, have found that any recurrence of a specific immune disease in a patient, in which the remission of the immune disease has been induced, can certainly be inhibited through the use of an agonist for sphingosine-1-phosphate receptor and have thus completed the present invention.
Accordingly, the present invention relates to an agent for maintaining remission after the remission induction therapy of a patient suffering from an autoimmune disease by the use of a biological agent or a nucleic acid synthesis-inhibitory agent, which comprises, as an effective component, an agonist for sphingosine-1-phosphate receptor.

### Effects of the Invention

The present invention would thus provide a method for the maintenance of the induced remission of an immune disease, which can significantly alleviate or reduce any serious side effect and likewise reduce the burdens exerted on the patients.

### Brief Description of the Drawings

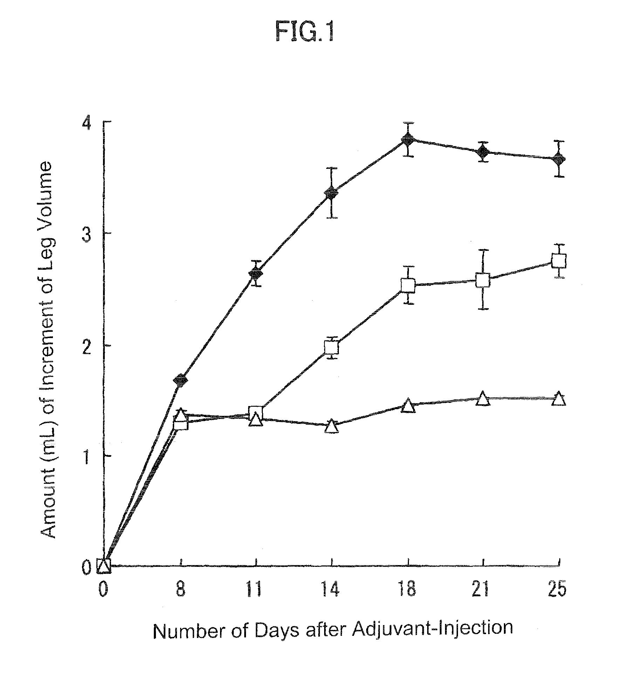

Figure 1 is a graph showing the effect (adjuvant-injected limb) of the switching over MTX to the compound 1, on a rat adjuvant-induced arthritis model. In this figure, the mark ◆ represents the results observed for the adjuvant-injected control animal group, the mark □ represents those observed for the animal group to which MTX was administered during the term extending from the first day (the 0^{th} day) to the 10^{th} day and the administration thereof to the test animals was discontinued on and after the 11^{th} day; the mark △ represents those observed for the animal group to which MTX was first administered during the term extending from the 0^{th} day to the 10^{th} day and then the compound 1 was administered to the test animals, in place of MTX, on and after the 11^{th} day (till the 24^{th} day).

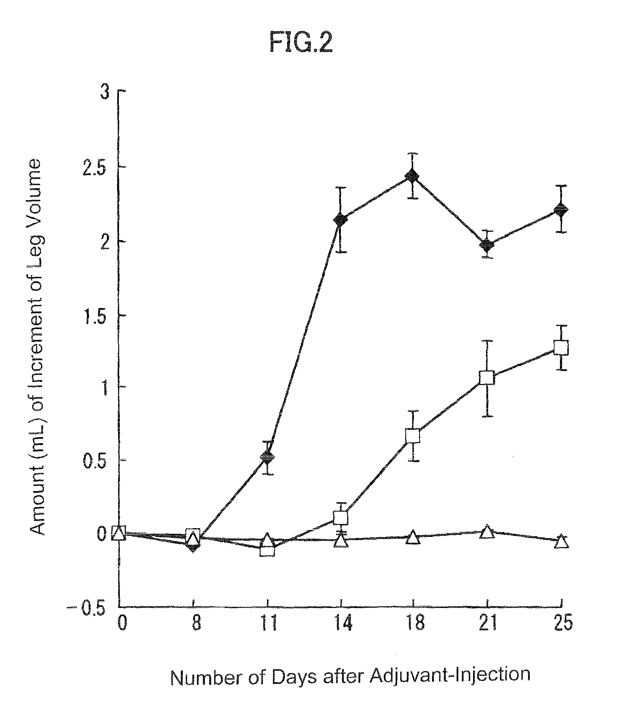

Figure 2 is a graph showing the effect (adjuvant injection-free limb) of the switching over MTX to the compound 1, on a rat adjuvant arthritis model. In this figure, the mark ◆ represents the results observed for the adjuvant-injected control animal group, the mark □ represents those observed for the animal group to which MTX was administered during the term extending from the first day (the 0^{th} day) to the 10^{th} day and the administration thereof to the test animals was discontinued on and after the 11^{th} day; the mark △ represents those observed for the animal group to which MTX was first administered during the term extending from the 0^{th} day to the 10^{th} day and then the compound 1 was administered to the test animals, in place of MTX, on and after the 11^{th} day (till the 24^{th} day).

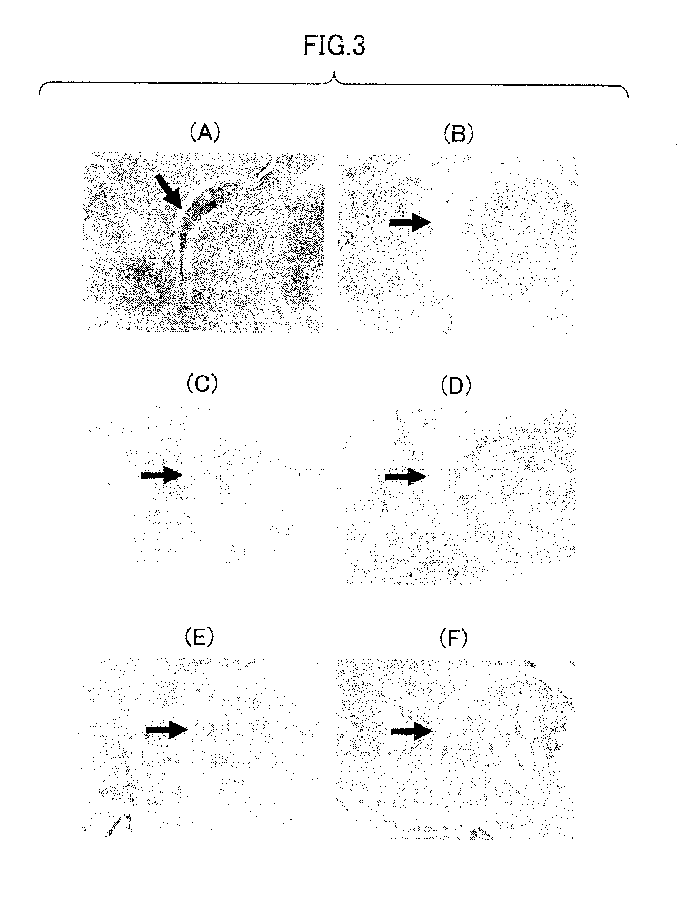

Figure 3 shows the images of the pathological tissues derived from the adjuvant-injected limb in the rat adjuvant-induced arthritis model. In this figure, the symbol A represents those observed for the adjuvant-injected control animal group (observed on the 11^{th} day); the symbol B represents those observed for the animal group to which MTX was administered during the term extending from the 0^{th} day to 10^{th} day (observed on the 11^{th} day); the symbol C represents those observed for the adjuvant-injected control animal group (observed on the 25^{th} day); the symbol D represents those observed for the animal group to which MTX was administered during the term extending from the 0^{th} day to 10^{th} day (observed on the 25^{th} day); the symbol E represents those observed for the animal group to which MTX was first administered during the term extending from the 0^{th} day to 10^{th} day and then MTX was switched over to the compound 1 (observed on the 25^{th} day); and the symbol F represents those observed for the normal control animal group. In addition, the arrow indicates the interior of the joint cavity.

### Mode for Carrying Out the Invention

In the present invention, the term "biological agent(s)" means an agent comprising a protein, an antibody or a peptide, which can show an effect of remission induction on an immune disease, and examples thereof include a soluble TNF receptor-fusion protein, an antibody or an interleukin receptor-antagonist. More specifically, there can be listed, for instance, a chimeric anti-TNF α -monoclonal antibody (infliximab), a soluble TNF receptor-fusion protein (etanercept), a human-type anti-TNF α-monoclonal antibody (adalimumab), a humanized anti-IL-6 receptor antibody (tocilizumab), an antagonist for IL-1 receptor, CTLA-4: Ig fusion protein (abatacept), and a chimeric anti-CD20 monoclonal antibody (rituximab).
The term "nucleic acid synthesis-inhibitory agent" used herein means a nucleic acid synthesis-inhibitory agent which shows an effect of remission induction on an immune disease (also including a agent capable of inhibiting the synthesis of nucleic acids through the inhibition of the synthesis of folic acid), and specific examples thereof include mizoribine, methotrexate and leflunomide.

The method for the remission induction, which makes use of these agents, can be implemented according to the procedures and/or the teachings disclosed in, for instance, "Manual for the Diagnosis of Rheumatoid Arthritis (Revised Edition): Manual for Diagnosis and Guideline for the treatment on the basis of EBM" (Juridical Foundation: the Endowment for Rheumatism of Japan, edited by OCHI Takahiro, 2004) and "Guideline for the implementation of TNF-Inhibitory Thereapy for Rheumatoid Arthritis (RA) (Revised Edition)", although the procedures to be selected may variously vary depending on the kinds of agents used.

It has been reported that the sphingosine-1-phosphate (S1P) simultaneously has an effect as an intracellular second messenger and an effect as an intercellular mediator in combination and that it is also involved in the transmission of information through a plurality of G protein conjugated type receptor (Endothelial Differentiation Gene: EDG) present on the surface of the cell membrane (see Non-Patent Document 1 and Non-Patent Document 3). There have presently been known five subtypes of S1P receptors, Edg-1, Edg-3, Edg-5, Edg-6 and Edg-8 and they are also referred to as S1P₁, S1P₃, S1P₂, S1P₄ and S1P₅, respectively. The compounds showing these S1P receptor agonist activities can exert effectiveness for a wide variety of diseases and accordingly, there have been reported various kinds of compounds having such activities.
The S1P receptor agonists used in the present invention may also be pharmaceutically acceptable salts thereof and they may likewise be those capable of showing such agonist activities through the phosphorylation with sphingosine-kinase enzyme.

Specific examples of such S1P receptor agonists include the compounds disclosed in WO 03/029184, WO 03/029205, WO 04/026817, WO 04/074297, WO 05/044780, WO 08/018427 and WO 08/018447. Preferred are, for instance, compounds represented by the following general formula and pharmaceutically acceptable salts thereof:

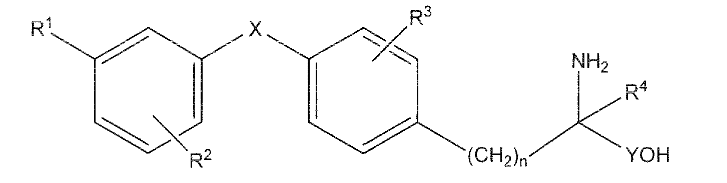

(In the foregoing general formula, R¹ represents a hydrogen atom, a halogen atom, a lower alkyl group having 1 to 4 carbon atoms which may be substituted with a halogen atom, a hydroxyl group, a phenyl group, an aralkyl group (in particular, a benzyl group or the like), a lower alkoxy group having 1 to 4 carbon atoms, a trifluoromethyloxy group, a phenoxy group which may have a substituent, a cyclohexylmethyloxy group, an aralkyloxy group which may have a substituent, a pyridylmethyloxy group, a cinnamyloxy group, a naphthylmethyloxy group, a phenoxymethyl group, a hydroxymethyl group, a hydroxyethyl group, a lower alkylthio group having 1 to 4 carbon atoms, a lower alkylsulfinyl group having 1 to 4 carbon atoms, a lower alkylsulfonyl group having 1 to 4 carbon atoms, a benzylthio group, an acetyl group, a nitro group or a cyano group;
R² represents a hydrogen atom, a halogen atom, a lower alkyl group having 1 to 4 carbon atoms, which may be substituted with a halogen atom, a lower alkoxy group having 1 to 4 carbon atoms, an aralkyl group or an aralkyloxy group;
R³ represents a hydrogen atom, a halogen atom, a trifluoromethyl group, a lower alkyl group having 1 to 4 carbon atoms, a lower alkoxy group having 1 to 4 carbon atoms, a hydroxyl group, a benzyloxy group, a phenyl group, a lower alkoxy methyl group having 1 to 4 carbon atoms or a lower alkylthio group having 1 to 4 carbon atoms;
R⁴ represents a hydrogen atom, a halogen atom, a lower alkyl group having 1 to 4 carbon atoms, a lower alkoxymethyl group having 1 to 4 carbon atoms, a lower alkylthiomethyl group having 1 to 4 carbon atoms, a hydroxymethyl group, a phenyl group or an aralkyl group;
X represents O, S, SO or SO₂;
Y represents -CH₂O-, -CH₂-, -CH=CH-, -CH=CF-, -FH₂CH₂-, -CF₂CFH-, -CH₂CF₂-, or -CH(OH)CF₂-; and
n represents an integer ranging from 1 to 4); or compounds represented by the following general formula or pharmaceutically acceptable salts thereof.

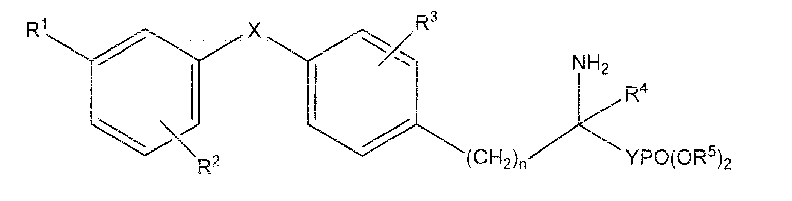

(In the foregoing general formula, R¹ represents a hydrogen atom, a halogen atom, a lower alkyl group having 1 to 4 carbon atoms which may be substituted with a halogen atom, a hydroxyl group, a phenyl group, an aralkyl group (in particular, a benzyl group or the like), a lower alkoxy group having 1 to 4 carbon atoms, a trifluoromethyloxy group, a phenoxy group which may have a substituent, a cyclohexylmethyloxy group, an aralkyloxy group which may have a substituent, a pyridylmethyloxy group, a cinnamyloxy group, a naphthylmethyloxy group, a phenoxymethyl group, a hydroxymethyl group, a hydroxyethyl group, a lower alkylthio group having 1 to 4 carbon atoms, a lower alkylsulfinyl group having 1 to 4 carbon atoms, a lower alkylsulfonyl group having 1 to 4 carbon atoms, a benzylthio group, an acetyl group, a nitro group or a cyano group;
R² represents a hydrogen atom, a halogen atom, a lower alkyl group having 1 to 4 carbon atoms, which may be substituted with a halogen atom, a lower alkoxy group having 1 to 4 carbon atoms, an aralkyl group or an aralkyloxy group;
R³ represents a hydrogen atom, a halogen atom, a trifluoromethyl group, a lower alkyl group having 1 to 4 carbon atoms, a lower alkoxy group having 1 to 4 carbon atoms, a hydroxyl group, a benzyloxy group, a phenyl group, a lower alkoxy methyl group having to 4 carbon atoms or a lower alkylthio group having 1 to 4 carbon atoms;
R⁴ represents a hydrogen atom, a halogen atom, a lower alkyl group having 1 to 4 carbon atoms, a lower alkoxymethyl group having 1 to 4 carbon atoms, a lower alkylthiomethyl group having 1 to 4 carbon atoms, a hydroxymethyl group, a phenyl group or an aralkyl group;
R⁵ represents a hydrogen atom or a lower alkyl group having 1 to 4 carbon atoms,
X represents O, S, SO or SO₂;
Y represents -CH₂O-, -CH₂-, -CH=CH-, -CH=CF-, -CH₂CH₂-, -CH₂CFH-, -CH₂CF₂-, or -CH(OH)CF₂-; and
n represents an integer ranging from 1 to 4).

The S1P receptor agonist is in general administered to a patient systemically or locally through oral route or parenteral route, but it is preferably orally administered since the burdens of the patient can be alleviated.

The dose of the S1P receptor agonist may vary depending on various factors such as the age, body weight and symptom of each individual patient as well as the therapeutic effect of the agent, the route of the administration thereof and the term of the treatment, but the S1P receptor agonist in a dose of 0.1 mg to 1,000 mg for the adult is administered once to several times a day when the receptor agonist is administered through the oral route, while the receptor agonist in a dose of 0.01 mg to 100 mg for the adult is administered once to several times a day when the receptor agonist is administered through the parenteral route.

When administering the S1P receptor agonist, it is used in the form of, for instance, a solid preparation for internal use and a liquid preparation for internal use, when it is administered through the oral route, as well as an injection, an external preparation and a suppository, when it is parenterally administered.

In the present invention, examples of the foregoing immune diseases include inflammatory bowel diseases, systemic lupus erythematosus, Crohn diseases, nephrotic syndromes, glomerular sclerosis, glomerulonephritis, multiple sclerosis, serious myasthenia, rheumatoid arthritis, psoriasis, allergic contact dermatitis, and atopic dermatitis.
In the present invention, the term "remission" means that each particular disease in itself is not completely cured, but the symptom (for instance, pain, swell and inflammatory reaction) thereof is temporarily or perpetually alleviated or disappeared.

As has been discussed above, the induction of the remission of an immune disease can be accomplished by the use of a biological agent or a nucleic acid synthesis-inhibitory agent in the early stage of the disease and then switching over the agent to the S1P receptor agonist sparingly having side effects after the remission induction. This accordingly permits the treatment of such an immune disease and/or the prevention of any recurrence of the disease, which is excellent in their effects, which infrequently show serious side effects and which are accompanied by reduced burdens exerted on the patient. In this respect, the term "switching" means that an S1P receptor agonist is used or administered to a patient in place of the biological agent or the nucleic acid synthesis-inhibitory agent and accordingly, the term does not include the simultaneous use of such a biological agent or a nucleic acid synthesis-inhibitory agent and an S1P receptor agonist in combination. This is because the switching is to prevent the occurrence of any side effect of the biological agent or the nucleic acid synthesis-inhibitory agent. Incidentally, when switching from a biological agent or a nucleic acid synthesis-inhibitory agent to an S1P receptor agonist, the biological agent or the nucleic acid synthesis-inhibitory agent may be temporarily used in combination with the S1P receptor agonist.

Thus, this treatment can not only inhibit the initiation of any systemic inflammation in a patient, but also inhibit the advancement of the inflammation reaction even at the site in which the symptom of the arthritis has been observed or detected.
While not being bound by any specific theory, the mechanism of action of the S1P receptor agonist in the present invention would be considered to be as follows: In the joint infiltrated by inflammatory cells such as sensitized T cells and/or macrophages, the biological agent which can inhibit the occurrence of any inflammation and the nucleic acid synthesis-inhibitory agent such as methotrexate act on the inflammatory cells which penetrate into the joint and thereby exclude the inflammatory cells from the interior of the joint. Thus, the advancement of the inflammation is prevented and therefore, the remission can be maintained. On or after the achievement of the remission in a patient, the S1P receptor agonist used in the present invention can inhibit any penetration of the inflammatory cells into the joint and can thus maintain the subsequent remission of the inflammation without using a biological agent or a nucleic acid synthesis-inhibitory agent. In other words, the remission of inflammation can effectively be maintained by the use of an S1P receptor agonist unless any such infiltration of inflammatory cells in the joint comes into existence.

### Examples

The present invention will be described in more detail below with reference to the following Examples, but the present invention is not restricted to these specific Examples at all.

### Example 1: Rat Adjuvant-Induced Arthritis Model

There were intracutaneously injected 0.05 mL (0.6 mg/animal) each of a suspension of killed M. butyricum (12 mg/mL) in liquid paraffin into the back of the right hind legs of 8-week-old female rats of LEW/CrlCrlj line (available from Japan Charles-River Co., Ltd.) to thus induce the arthritis (the 0^{th} day). Then 0.5 mL (per body weight of 100 g) each of an agent solution prepared using a 0.5% methyl cellulose (0.5% MC) was orally administered to each of the test animals. To the adjuvant-injected control animal group, there was administered only the 0.5% MC. Methotrexate (MTX, available from Sigma Company) (0.1 mg/kg) was administered to each test animal, once a day, during the term extending from the 0^{th} day to the 10^{th} day, every day. On or after the 11^{th} day, (R)-2-amino-5-[2-chloro-4-(3-trifluoro methylphenylthio)phenyl]-2-methyl-1-pentanol hydrochloride (hereunder simply referred to as "Compound I") (1 mg/kg) was administered to the test animals, instead of methotrexate, once a day up to the 24^{th} day every day. The evaluation of the arthritis was carried out according to the following procedures: The volumes of the right and left hind legs of each test animal were determined on the 0^{th}, 8^{th}, 11^{th}, 14^{th}, 18^{th}, 21^{st} and 25^{th} days of the testing period using a volume-determining meter (MK-550 available from Muromachi Machine, Co., Ltd.) and then the rate of increase in the volume of the hind leg was determined for each of the right and left hind legs of each individual test animal. Then there was prepared a pathological specimen (stained with hematoxylin eosin) of a joint extending from the distal end of the shinbone to the tarsalia to evaluate the degree of the infiltration of inflammatory cells into the joint.

In case of the adjuvant-induced arthritis, acute inflammation, whose far advanced stage is observed on around 3^{rd} day, is recognized on the adjuvant-injected leg and thereafter it is once degenerated. Then the systemic inflammation due to the systemic immunological abnormality is initiated on about 10^{th} day and as a result, the onset of arthritis is observed on the non-injected leg in parallel with the injected leg.
In case of the injected leg, the symptom of arthritis due to the adjuvant injection was recognized on the 8^{th} day, the systemic inflammation was initiated on and after the 11^{th} day and the symptom of arthritis was further advanced (see the data plotted on Fig. 1). In case of the animal group to which MTX was administered during the term extending from the 0^{th} day to the 10^{th} day, there was not observed any increase in the volume of the leg and the progress of the arthritis was found to be inhibited during the term extending from the 8^{th} day to the 11^{th} day, but when stopping the administration of MTX, the symptom of arthritis was again advanced (see the data plotted on Fig. 1). In case of the animal group to which MTX was administered during the term extending from the 0^{th} day to the 10^{th} day to suppress the onset of the arthritis and then MTX was switched over to Compound 1, the progress of the arthritis was inhibited (see the data plotted on Fig. 1).

In the non-injected leg, the symptom of the arthritis was recognized from about 11^{th} day (see the data plotted on Fig. 2). In the animal group to which MTX was administered during the term extending from the 0^{th} day to the 10^{th} day, the onset of the arthritis in the non-injected leg was suppressed at the time of the 11^{th} day, but the arthritis was initiated when stopping the administration of MTX (see the data plotted on Fig. 2). In case of the animal group to which MTX was first administered during the term extending from the 0^{th} day to the 10^{th} day to thus suppress the onset of the arthritis and then MTX was switched over to Compound 1, the onset of the arthritis was suppressed (see the data plotted on Fig. 2).
Thus it was found that MTX could suppress the onset and progress of arthritis, but the arthritis was immediately advanced when stopping the administration thereof. Compound 1 as an S1P receptor agonist could maintain the MTX-suppressed condition of the symptom of the arthritis.

Fig. 3 shows the images of the pathological tissues of the joint extending from the distal end of the shinbone to the tarsalia. In case of the adjuvant-injected control animal group, there were recognized, in the injected legs, the conspicuous infiltration of inflammatory cells into the joint on the 11^{th} day (see Fig. 3A) and the 25^{th} day (see Fig. 3C). In the animal group to which MTX was administered during the term extending from the 0^{th} day to the 10^{th} day, any infiltration of inflammatory cells into the joint was suppressed at the time of the 11^{th} day (see Fig. 3B), When stopping the administration of MTX, however, the conspicuous infiltration of inflammatory cells into the joint was recognized (see Fig. 3D). In case of the animal group to which MTX was first administered during the term extending from the 0^{th} day to the 10^{th} day to thus suppress the progress of the arthritis and then MTX was switched over to Compound 1, any infiltration of inflammatory cells into the joint was suppressed (see Fig. 3E).

Incidentally, even in case of the non-injected legs, any infiltration of inflammatory cells into the joint was found to be suppressed in the animal group to which MTX was first administered during the term extending from the 0^{th} day to the 10^{th} day to thus suppress the onset of the arthritis and then MTX was switched over to Compound 1.

### Industrial Applicability

The present invention provides a method for maintaining the induced remission of an immune disease, which can alleviate or reduce any side effect and the burdens exerted on the patient suffering from the arthritis.

## Claims

1. An agent for maintaining induced remission after the remission induction for an immune disease by the use of a biological agent or a nucleic acid synthesis-inhibitory agent, comprising, as an effective component, an agonist for sphingosine-1-phosphate receptor.

2. The agent for maintaining induced remission as set forth in claim 1, wherein the biological agent is a soluble TNF receptor-fusion protein, an antibody or an interleukin receptor-antagonist.

3. The agent for maintaining induced remission as set forth in claim 1, wherein the nucleic acid synthesis-inhibitory agent is mizoribine, methotrexate and leflunomide.

4. A method for maintaining induced remission comprising carrying out the remission induction for an immune disease by the administration of a biological agent or a nucleic acid synthesis-inhibitory agent to a patient suffering from the disease and then administering an agonist for sphingosine-1-phosphoric acid receptor to the patient.

5. Use of an agonist for sphingosine-1-phosphate receptor for the preparation of an agent for maintaining induced remission, said agent being used after the remission induction for an immune disease by the administration of a biological agent or a nucleic acid synthesis-inhibitory agent to a patient suffering from the disease.
